Europäisches Patentamt

European Patent Office

Office européen des brevets

(19)

(11) Publication number: **0 214 601**
A2

(12)

# EUROPEAN PATENT APPLICATION

(21) Application number: 86112124.2

(22) Date of filing: 02.09.86

(51) Int. Cl.⁴: **A 01 H 1/06**
**C 12 N 15/00**

(30) Priority: 04.09.85 US 772314

(43) Date of publication of application:
18.03.87 Bulletin 87/12

(84) Designated Contracting States:
AT BE CH DE FR GB IT LI LU NL SE

(71) Applicant: DNA PLANT TECHNOLOGY CORPORATION
(under the laws of the state of Delaware)
2611 Branch Pike
Cinnaminson, New Jersey(US)

(72) Inventor: Bravo, Janis Egli
431 Cinnaminson Avenue
Palmyra New Jersey(US)

(72) Inventor: Evans, David Alan
917 Lincoln Avenue
Palmyra New Jersey(US)

(74) Representative: Patentanwälte Grünecker, Kinkeldey,
Stockmair & Partner
Maximilianstrasse 58
D-8000 München 22(DE)

(54) Creation of cytoplasmic male sterility maintainer line trough protoplast fusion.

(57) This invention relates to a process for perpetuating a cytoplasmic male sterile line of a plant species capable of regeneration which comprises:

a) combining a nucleus from a protoplast derived from the cytoplasmic male sterile line with cytoplasm from a protoplast derived from a male fertile line under protoplast fusion conditions, to form a male fertile cybrid;

b) culturing and regenerating the cybrid under culturing and regeneration conditions to produce a male fertile adult plant;

c) crossing the male fertile plant with a plant of the cytoplasmic male sterile line so that cytoplasmic male sterile progeny are produced.

# CREATION OF CYTOPLASMIC MALE STERILITY
## MAINTAINER LINE THROUGH PROTOPLAST FUSION

The present invention relates to a method of creating a cytoplasmic male sterile maintainer line; more specifically, it relates to a method of producing a maintainer line by protoplast fusion.

Plant breeders have long been attempting to increase productivity of the more important crops used for food, or for processing as feed, fiber and pharmaceuticals, by their efforts in developing cultivars (cultivated varieties) with particularly desirable characteristics. One of the ways in which this aim is frequently accomplished is the development of superior plant lines by infusing desirable traits with already existing cultivars, thus potentially forming a hybrid with exceptional characteristics. The general superiority of $F_1$ hybrids over either of their parents is a widespread phenomenon in a variety of different types of crops. This superiority may express itself in such features as increased height, growth rate, leaf area, early flowering and overall higher yields.

One way in which the production of superior plant lines has been achieved in the past is by the making of numerous manual cross pollinations to obtain the desired $F_1$ hybrid. These crosses generally are carried out between an already existing cultivated crop variety and an unadapted or "wild type" gene donor which possesses one or more traits which the breeder wishes to incorporate into the cultivated variety. Once the production of the $F_1$ has been accomplished, repeated backcrossings and selections are then required to ultimately obtain a plant containing all the characteristics of the cultivated plant as well as retaining the new, desirable traits introduced from the "wild type" plants. As can easily be seen, this selection procedure is extremely tedious and time consuming; yet, in spite of the difficulties, it remains one of the most widespread of plant breeding techniques currently in use.

Because of the problems involved with this method, a number of other avenues for more efficient production of $F_1$ hybrids have been and are being explored. Among the most avidly pursued fields of endeavor is the construction of male sterile lines within the varieties of crop plants to which hybridization is desired. The principle behind the development of male sterile lines is that, in order to produce hybrid seed more economically, the restrictions of controlled cross-fertilization imposed by floral morphology, especially of perfect flowers, must be overcome. To this end, the female parent should be prevented from self-or intraline fertilization. The elimination of self-fertilization requires male sterility, for example the inability of the plant to produce viable pollen. The establishment of the male sterile line thus renders any crop variety readily adaptable to hybridization with virtually any gene donor having the desired characteristics, and eliminates the need for laborious hand pollination.

Male sterile lines may be established in a number of ways. Hand emasculation is one method by which a line may be sterilized. For example, large scale production of hybrid corn may be done by detasselling the female parent; however, the large scale emasculation of species having perfect flowers generally proves to be economically unfeasible.

Genetic male sterility is also a known trait, usually inherited as a recessive and monogenic trait in a number of different types of plants. Exploitation of this characteristic is used to produce hybrid seed of barley, tomato, pepper, marigold, zinnia, and others. However, there is a basic shortcoming in the use of this technique, in that it is difficult to obtain a 100% genetic male sterile stand. Overcoming this difficulty requires a rather complex use of clever genetic manipulation. Its use, therefore, is currently restricted to hybrid seed production of cultivated plants in which cytoplasmic male sterility has not been found, or that in which the male sterile plasmatype exhibits inferior agronomic performance.

Cytoplasmic male sterility provides an additional mechanism for providing the desired lines for use in hybridization. In this situation, the genetic factors controlling male sterility are found in the cytoplasm. It is generally believed that the trait is associated with genes found in the mitochondria. The use of cytoplasmic male sterile lines has rapidly found application in the production of hybrid seed. Widespread production based on this trait is responsible for higher yields of many important cultivated crops such as sorghum, sugarbeet, onions, melon, and, most successfully, corn. In spite of the popularity of this method, however, there are still a number of difficulties associated with perpetuating the male sterile lines once they are obtained. Generally, once the cytoplasmic male sterile (CMS) line is produced, it must be maintained by a series of backcrosses. Needless to say, the procedure is quite time consuming and difficult; however, a further problem exists in that the integrity of the nuclear genes of the CMS line cannot be guaranteed unless a male fertile line with an identical nucleus can be created. Thus, although the use of CMS lines to create hybrid seed is quite useful and popular, a simpler method of maintaining the created CMS line is clearly desirable if the technique is to be exploited most efficiently.

In recent years, because of the potential importance of the CMS trait in the production of hybrid seed, a great deal of research has been devoted to the mechanisms involved in the trait, and the patterns of distribution of the organelles controlling it. For example, Flick, et al. (Biotechnology 3: 555-559, 1985) have described protoplast fusions to produce cybrids between different species of Nicotiana, and observed the pattern of segregation of organelle traits, of which CMS is but one, in the plants regenerated from the cybrid fusion products. Similarly, Aviv, et al. (Theoret. Appl. Genet. 58: 121-127, 1980) have also performed protoplast fusions between different species of Nicotiana, using x-irradiated cells to determine the possibility of transferring organelle-associated traits between species. Numerous other authors have also reported somatic hybridization between different species of various plants to transfer the CMS trait. Yet, even in light of the tremendous increase in knowledge of the patterns of segregation of organellar traits, consideration of the new information has been on a highly theoretical level, and no practical application, nor any commercial varieties have yet been made which would aid in the more efficient commercial utilization of the CMS trait in plant breeding.

It has now been unexpectedly discovered that cytoplasmic male sterile maintainer lines may be created without the need for extensive backcrossing. In fact, the present invention provides a method of creating a maintainer line essentially in a single step. This is achieved by a process of protoplast fusion in which the cytoplasm of a protoplast from a male fertile line is combined with the nucleus from a protoplast of the CMS line. The resulting cytoplasmic hybrid, or cybrid, will thus possess the nuclear genome of the desired male sterile line, but will also be fertile. The newly produced male fertile "maintainer" line produced by regeneration of the fusion product into an adult plant may then be crossed with a male sterile which acts as the female parent; because of maternal inheritance of cytoplasm, the resulting progeny will all be male sterile. Thus, the male sterile line is easily propagated without backcrossing, and also without the attendant danger of loss of the integrity of the desirable nuclear traits of the CMS line. This therefore represents a significant improvement over previously known techniques for maintaining cytoplasmic male sterile plant lines.

The present invention relates to a method for propagating a cytoplasmic male sterile line of a species capable of regeneration which comprises combining, by protoplast fusion, a nucleus from a protoplast of a cytoplasmic male sterile line with cytoplasm of a male fertile line to form a male fertile cybrid, regenerating the cybrid into a male fertile adult plant; and crossing the male fertile plant with a plant from the cytoplasmic male sterile line, to obtain cytoplasmic male sterile progeny. It further relates to above process of protoplast fusion to produce the maintainer line, to be used in propagation. It also relates to the maintainer line so produced, and the male sterile progeny produced by the cross.

As noted above, cytoplasmic male sterile lines require, in order that the desirable traits present be perpetuated beyond a single generation, that a fertile maintainer line be established to produce more of the CMS line. This has traditionally been accomplished by crossing the maintainer line, which is ideally isogenic with the male sterile line, except for the sterility trait, with the CMS line to yield male sterile offspring. The maintainer line of the present invention operates in essentially the same manner, but eliminating the necessity for achieving a line isogenic with the male sterile line.

The maintainer is produced by a process of protoplast fusion in which the cytoplasmic male sterile nucleus is combined with the cytoplasm of a male fertile line. In this procedure, the protoplasts, plant cells from which the cell wall has been removed, must first be obtained. Isolation of protoplasts may be achieved by a number of known techniques. Generally, this is done by enzymatic treatment of various types of plant tissue in order to produce the protoplasts. Among the commonly used enzymes are cellulases, hemicellulases and pectinases. Although the enzyme or enzymes employed will vary depending upon the plant tissue being used, it is within the knowledge of the skilled technician to determine which treatment is most appropriate for his purposes. A number of techniques for protoplast isolation and culture are well documented in the scientific literature; a summary of many of the known procedures is found in Handbook of Plant Cell Culture, Vol. I, Chapter 4, Tables 4 and 7, (Evans, et al., eds.), 1983.

Rarely, isolated protoplasts will fuse spontaneously. It is more often the case that chemical treatment is required to induce the desired levels of protoplast fusion. The most common method of promoting fusion is the addition of polyethylene glycol(PEG) to the protoplast mixture. Treatment with PEG causes agglutination of the protoplasts, which then fuse during elution in the presence of calcium and high pH, preferably at least 10. Most frequently, the PEG used has a molecular weight of 1540, although this may be varied within a broad range. Examples of typical solutions needed for the process of protoplast fusion may be found in Table 1. Fusion utilizing PEG, although not the only useful method, is particularly preferred because it is non-specific, and has been successfully used to produce both inter- and intraspecific hybrids. A typical protocol for protoplast fusion, according to the technique described by Kao and Michayluk (Planta 115: 355-367, 1974) is presented in Table 2. As already noted, other chemicals such as polyvinyl alcohol and various PEG derivatives, have been shown to be useful in inducing protoplast fusion, but best results are achieved when PEG is used.

A number of possible fusion products may be obtained by the above method. One possibility is that the nuclei of the two separate lines may fuse, producing a somatic hybrid. In most cases, in connection with the present invention, this is an undesirable result, since it will defeat the desired result of maintaining the integrity of the CMS genome. Therefore, the preferred products for the present purposes are cybrids, fusion products in which no nuclear fusion has occurred. Because it is, superficially at least, impossible to visually distinguish which of the resulting protoplasts are actually fusion products, and further which are cybrids rather than hybrids, it is desirable to incorporate some sort of "marker" system into the procedure. Separation of fusion products from parental protoplasts can be accomplished relatively easily, if tediously, by utilizing parental protoplasts which are morphologically distinct. For example, fusion of chlorophyll containing protoplasts with colorless cell culture protoplasts containing starch granules yields a readily identifiable fusion product. Identification of fusion products is not so critical, however, as is cybrid identification. This is most readily done by exploiting the occurrence of genetic complementation in order to identify and eliminate those protoplasts in which nuclear fusion has occurred. For example, a number of plant species exhibit nuclear-based chlorophyll defects which cause the plant to be albino when present alone. However, when this trait is present in conjunction with genetic information for normal coloration, the resulting plant will often be lighter green in color than normal, readily identifying those plants derived from protoplasts in which nuclear fusion has occurred. Similarly, use of a mutant plant which exhibits resistance to certain antibiotics such as a lincomycin or streptomycin is also possible. Since a wide variety of such traits exist in a number of different plant species (see Table 3), it is within the ability of the skilled artisan to determine which trait may be usefully employed as a marker, given the genetic makeup of the CMS parent. The additional important event, the fusion of the male fertile cytoplasm with a CMS protoplast, is of course easily verified by observing whether the resulting regenerated plants are male fertile or male sterile. Observation of _fertility_ in regenerated plants, combined with the appearance of the selected nuclear marker trait, immediately identifies those plants derived from protoplasts in which the required fusion of CMS nucleus with fertile cytoplasm has occurred. These mature plants thus represent the desired maintainer line. These plants can then be used as the male parent in a cross with the original CMS plant line, to produce progeny all of which, because of strict maternal inheritance of cytoplasm, will be cytoplasmic male sterile.

The foregoing procedure may be successfully performed with a wide variety of plants. It is possible to fuse protoplasts from any two species, and even a number of viable intergeneric fusion products are known. Generally speaking, regardless of the type of plant used, the fusion procedure will not be the process limiting step. The primary limitation in application of the process is that the fused protoplast must be capable of regeneration into an adult plant. In most circumstances, greater success in regeneration is observed when the two cell lines are of closely related species; this will thus to some extent limit the possible choices for a cytoplasm donor, which will preferably be in the same genus as the CMS plant.

The present procedure is particularly amenable to use with members of the family Solanaceae, which includes such important crop plants as tomato, potato, pepper, eggplant and tobacco. This group of plants has shown the greatest success in regeneration of plants from protoplasts. To date, at least 40 species of this family have been regenerated from cultured protoplasts. The genus _Nicotiana_ is particularly well suited to use in cell culture techniques. However, regeneration is by no means limited to this family. In fact, a wide variety of non-solanaceous species, such as carrot, cabbage and asparagus, among others, have been successfully regenerated from protoplasts; recovery of callus from protoplast culture, indicating potential amenability to regeneration, has also been observed in such economically important species as turnip, soybean, coffee and rice. A partial list of species in which regeneration has been accomplished is presented in Table 4. It will be readily apparent to the skilled artisan that the present procedure is not limited to practice with these species in which regeneration is now known, but is also applicable to any species for which a regeneration procedure is ultimately developed.

The details of the regeneration procedure will vary depending upon the plant species used. In general terms, the fusion product protoplasts must first be cultured in order to allow redevelopment of the cell wall and to induce cell division. Typically, the protoplast culture requirements are simply modifications of known plant cell culture. Most commonly used growth media are B5 and MS media; however, carbon sources and nutrients may be varied to obtain optimum growth. Growth regulators are also added to the culture medium to stimulate cell division. Generally, but not universally, both an auxin and a cytokinin are used in the initial culture medium, but the concentration of the hormones varies depending on the plant species used. There are also a variety of techniques, such as droplet culture, agar cultures, and hanging droplets, by which the actual culture may be achieved. The techniques involved in culture of protoplasts of different plant types are widely reported in the scientific literature, and it is within the ability of the skilled artisan to modify the known techniques to suit the desired end.

Once rapidly growing callus has been established in the protoplast culture, callus is then recovered from the culture medium, and transferred to a regeneration medium. The regeneration medium used is to a large extent species specific, and is generally based on the conditions necessary to achieve plant regeneration from callus of the particular species used. Although temperature is not usually critical, optimum light requirement may differ dramatically between species. Again, however, the conditions required for various speices are widely reported and readily available to one skilled in the art. For a partial summary of known regeneration protocols, and reference sources, for example, see Evans, et al., eds. Handbook of Plant Cell Culture, Vol. 1, Chap. 4, Table 7, p. 146-154, 1983.

The result of successful regeneration is the production of the required maintainer lines. As noted above, the plants derived from protoplasts which represent the desired combination of CMS nucleus and fertile cytoplasm are readily identified by the presence of the marker characteristics incorporated into the system, combined with male fertility. The resulting plants may then be used in a typical breeding program with the established CMS lines to produce the required CMS progeny.

The above description presents an overview of the general procedures required to establish maintainer lines and their use in the propagation of established cytoplasmic male sterile lines. The process of the present invention may be better understood by reference to the following non-limiting examples.

## EXAMPLE 1

This example demonstrates the process of protoplast isolation, utilizing a cytoplasmic male sterile Nicotiana tabacum produced by repeated backcrossing; this line contains the nucleus of N. tabacum and the cytoplasm of N. repanda. As the male fertile cytoplasm donor, an N. tabacum Su/Su mutant, a semidominant homozygous albino, was used.

For isolation of protoplasts from the CMS plant, the plant to be used is first placed in the dark for 24 hours. It is preferred to use a plant prior to flowering. The youngest, fully expanded leaves are sterilized in 8% Clorox for 10 minutes, then revised three times with sterile, distilled water. The sterilized leaves are then left to air dry under sterile conditions.

After drying, the lower epidermis is peeled from the leaf with fine dissecting forceps (Ladd Instrument Corp.). The peeled leaf is then cut into sections, and about 0.5 g of leaf tissue is transferred, peeled side down, into a 60 x 15 mm petri dish containing the following enzyme mixture.

| | |
|---|---|
| Cellulase | 0.5% |
| Macerase | 0.5% |
| Driselase | 0.1255% |
| dissolved in KM medium 8P, with | |
| pH adjusted to 5.5-5.8 | |

The peeled leaf in enzyme solution is incubated in the dark, with occasional shaking, until protoplasts are released, usually about 4-5 hours. The resulting solution contains protoplasts as well as cell fragments and undigested leaf material. The mixture is then purified to isolate the protoplasts.

Similarly, protoplasts from a Su/Su mutant are also obtained. In this case, however, cells are removed from a rapidly growing suspension culture. Such cell suspensions are maintained by subculturing every 3-4 days in MS medium containing 4.5 uM 2,4-D 2.0 ml of the culture is combined in a 60 X 15 mm petri dish containing an enzyme mixture of the following composition:

| Cellulase | 2.0% |
|-----------|------|
| Macerase | 1.0% |
| Driselase | 1.0% |

dissolved in enzyme isolation solution (EIS) containing 0.7 M glucose, 3.0 mM MES, 6.0 mM $CaCl_2 \cdot H_2O$ and 0.7 mM $NaH_2PO_4-H_2O_1$ pH 5.6-5.8.

The mixture is incubated in the dark while shaking (30-50 rpm) for 5 hours. The resulting mixture contains some undigested cell material, and is purified before further use.

The purification of protoplasts in each case is performed as follows:

The protoplast-enzyme mixture is passed through a stainless steel mesh with a pore size of 67 um, to separate protoplasts from undigested cells. The enzyme is then washed from the protoplasts by centrifuging at 40 x g in a sterile conical test tube.

The supernatant is decanted and discarded using a sterile pasteur pipette. The pelleted protoplasts are resuspended in 4.0 ml of 8p medium (as described in Kao and Michayluk, Planta 126: 105-110, 1975). Centrifugation and decantation is repeated. The washed protoplasts are then resuspended in 8p medium (2.0 ml medium per 0.5 ml of protoplast pellet).

-14-  0214601

The protoplasts are then ready for protoplast fusion, performed in accordance with the protocol described in Table 2.

### EXAMPLE 2

The following example describes a procedure for culture and regeneration of fused protoplasts:

Division of protoplasts begins within 2-5 days. Fresh Medium 8p is added to droplets every 1-2 weeks until a rapidly proliferating callus is visible. Shoot regeneration from callus is then achieved by adding MS medium (Murashige and Skoog, Physiol Plant 15: 473-497, 1962) containing 5.0 uM 6-Benzyl adenine 6BA to the liquid protoplast-derived suspension at two week intervals. Shoots generally regenerate within 6 weeks.

The regenerated shoots are rooted by transferring to half-strength MS with agar containing 0.11 uM 3-aminopyridine. When an extensive root system has formed, the regenerated shoots are transferred to peat pellets under high humidity for further growth. As the regenerated plants mature, the humidity is gradually decreased, and the light intensity gradually increased, before transfer of the regenerated plants to greenhouse conditions.

## EXAMPLE 3

The plants regenerated according to Example 2 are then examined to determine which are suitable for use as maintainer lines. The fate of the nuclear genes are followed by observing the color of the leaves of the regenerated plants. Because the Su/Su parent was a semi-dominant homozygous albino, if nuclear fusion had occurred, leaf color will be light green. In the present series of experiments, all regenerated plants turned out to be dark green, indicating that no nuclear fusion had occurred. All plants additionally proved to have 48 (2N) chromosomes as did the sterile parent.

Cytoplasmic transfer is followed by observing whether flowers of the regenerated plants were male fertile or male sterile. Both types were in fact recovered among the regenerated plants. Cytoplasmic transfer had occurred in those plants which were dark green and male fertile (2n-48). Each of these plants has the nucleus of the male sterile parent and the cytoplasm of the male fertile plant, and constitute the appropriate CMS maintainer line.

The original CMS line and the maintainer lines are grown to maturity under greenhouse conditions. At maturity, the pollen produced by the fertile maintainer line is used to fertilize the CMS plants. Once pollination of the CMS plants has been accomplished the pollinated flowers are covered to prevent any other pollen from coming into contact with them. Seeds are ready for collection within one month of pollination. All seeds collected from the CMS line produce CMS progeny.

TABLE 1:   Typical Protoplast Fusion Solutions

Enzyme Wash Solution

    Dissolve:  0.5 M Sorbitol (9.1 g)

               5.0 mM $CaCl_2 \cdot 2H_2O$ (75 mg)

    in 100 ml final volume.        pH = 5.8


PEG Fusing Solution

    Dissolve:  0.2 M Glucose (1.8 g)

               10 mM $CaCl_2 \cdot 2H_2O$ (73.5 mg)

               0.7 mM $KH_2PO_4$ (4.76 mg)

    in 50 ml final volume.

    To this 50 ml, add 25 g of PEG

    and dissolve.                 pH = 5.8


PEG Eluting Solution

    Dissolve:  50 mM Glycine (375 mg)

               0.3 M Glucose (5.4 g)

               50 mM $CaCl_2 \cdot 2H_2O$ (735 mg)

    in 100 ml final volume.       pH = 10.5 using NaOH pellets.


Alternately, this solution can be prepared as two solutions
that are mixed at the time of fusion.

    (A)    100 mM Glycine (750 mg)

           0.3 M Glucose (5.4 g)

    in 100 ml final volume.       pH = 10.5 using NaOH pellets.

    (B)    100 mM $CaCl_2 \cdot 2H_2O$ (1470 mg)

           0.3 M Glucose (5.4 g)

    in 100 ml.


Using these two solutions permits storage without visible
precipitation.  Mix A with B in 1:1 ratio at time of fusion

TABLE 2: Polyethylene Glycol Method of Protoplast Fusion

1.  0.5 ml of protoplasts from two sources are mixed and diluted to 8 ml with enzyme wash solution (Table 6).  This mixture is centrifuged at 100 g for 4 minutes.

2.  Excess enzyme solution is decanted and the mixed protoplasts are resuspended in enzyme wash solution. Steps 1 and 2 are repeated once.  After the second decantation, the precipitated protoplasts are resuspended in 1.0 ml of enzyme wash solution.

3.  Put one drop of silicone fluid (Sigma Chemical Company) in a Falcon (1007) petri dish (60 x 15).  Place a 22 x 22 mm coverslip on top of the silicone drop.

4.  Pipette 0.15 ml of mixed protoplasts onto the glass coverslip.  Allow the protoplasts to settle on the coverslip for ca. 5 minutes to form a thin layer of protoplasts.

5.  Carefully add 0.45 ml of PEG solution (Table 6) to the protoplast mixture.  To create a uniform single layer of protoplasts attached to the coverslip, the PEG should be added to one side of the protoplast droplet.

6.  Incubate the protoplasts in the PEG solution at room temperature for 15-20 minutes.

7.  Add 0.9 ml of PEG eluting solution to the mixture.  After 10 minutes, add an additional 0.9 ml to the mixture.

0214601

8. After the second elution treatment, a protoplast culture medium is added to the protoplasts to aid in removal of the PEG and the eluting solution.

9. Add 0.5 ml of culture medium to the mixture. An additional 0.5 ml is added after 10 minutes.

10. After the final wash, the protoplasts should be in 1-2 ml of culture medium. The petri dish can be sealed with a double layer of parafilm and examined in inverted microscope to ascertain the frequency of fusion.

TABLE 3: Phenotypes of Cells Used for Complementation or
Partial Complementation to Recover Somatic Hybrid Cells or
Plants

| PATENT 1 | PARENT 2 | HYBRID | GENUS |
|---|---|---|---|
| Chlorophyll defect (s) | Chlorophyll defect (v) | Green | Nicotiana |
| Chlorophyll defect | Chlorophyll defect | Green | Datura |
| Chlorophyll defect (Su/Su) | Normal | Light Green | Nicotiana |
| 5MT resistant | AEC resistant | 5MT resistant, AEC resistant | Nicotiana |
| NR defect (nia) | NR defect (cnx) | Normal | Nicotiana |
| NR defect (nia) | Chlorophyll defect | Normal | Nicotiana |
| Kanamycin | Unable to regenerate | Kanamycin | Nicotiana |
| Actinomycin | Actinomycin | Actinomycin | Petunia |
| Streptomycin | Normal | Streptomycin | Nicotiana |
| Streptomycin | Auxin autotrophic | Streptomycin, auxin auto-trophic | Nicotiana |

[a]NR= Nitrate Reductase; 5MT=5 Methyl tryptophan; AEC =
Aminoethyl cysteine.

TABLE 4: Partial List of Non-Solanaceous Species which have been Successfully Regenerated from Protoplasts; ( ) = Source of Protoplast

<u>Species</u>

<u>Antirrhinum majus</u>
   (mesophyll)

<u>Arabidopsis thaliana</u>
   (suspension)

<u>Asparagus officinalis</u>
   (cladodes)

<u>Brassica napus</u>
   (mesophyll)

<u>B. napus</u>
   Haploid
   (mesophyll)

<u>Cichorium endiva</u>
   <u>C. intybus</u>
   <u>Gaillardia grandiflora</u>
   <u>Helianthus annuus</u>
   <u>Nigella arvensis</u>
   <u>Senecio jacobea</u>
   <u>S. silvaticus</u>
   <u>S. viscosus</u>
   (shoot tip or
   mesophyll)
<u>Daucus carota</u>
   (suspension)
<u>D. carota</u> (root)

<u>B. napus</u>
<u>B. oleracea</u>
   Cabbage
   (roots)

<u>B. napus</u>
   Haploid
   (mesophyll)

<u>B. napus</u>
   Haploid
   (stem embryo)

<u>Bromus inermis</u>
   (suspension)

<u>Citrus sinensis</u>
   (ovule callus)

<u>Hemerocallis spp.</u>
   (suspension)

<u>Manicot esculenta</u>
   (mesophyll)

<u>Medicago sativa</u>
   (mesophyll)

<u>Medicago sativa</u>
   (mesophyll)

TABLE 4 (Continued)

D. carota
(callus)

D. carota
(suspension-
proembryo
aggregates)

Digitalis lanata
(mesophyll)

D. purpurea
(suspension)

Nemesia strumosa
(mesophyll)

Panicum maximum
(suspension)

Pelargonium sp.
(mesophyll)

Pennisetum americanum
(suspension)

Ranunculus sceleratus
(mesophyll)

Senecio vulgaris
(mesophyll, callus)

Trifolium repens
(suspension)

What is Claimed is:

1. A process for perpetuating a cytoplasmic male sterile line of a plant species capable of regeneration which comprises:

a) combining a nucleus from a protoplast derived from the cytoplasmic male sterile line with cytoplasm from a protoplast derived from a male fertile line under protoplast fusion conditions, to form a male fertile cybrid;

b) culturing and regenerating the cybrid under culturing and regeneration conditions to produce a male fertile adult plant;

c) crossing the male fertile plant with a plant of the cytoplasmic male sterile line so that cytoplasmic male sterile progeny are produced.

2. The process of Claim 1 wherein the species is a member of the Solanaceae.

3. The process of Claims 1 or 2 wherein the species is a member of the genus Nicotiana.

4. The process of any of Claims 1-3 wherein the species is Nicotiana tabacum.

5. The process of any of the Claims 1-4 wherein protoplast fusion is induced by PEG, in the presence of calcium and a pH of at least 10.

6. In a process for producing maintainer lines for cytoplasmic male sterile plant lines of a plant species capable of regeneration which comprises creating a male fertile line by restoring fertility to a male sterile line, the improvement comprising creating the male fertile line by combining, under protoplast fusion conditions, a nucleus from a protoplast derived from a male sterile line with cytoplasm derived from a protoplast of a male fertile line to create a male fertile cybrid, and regenerating from the cybrid an adult male fertile maintainer plant.

7. The process of Claim 6 wherein the species is a member of the Solanaceae.

8. The process of Claim 6 or 7 wherein the species is a member of the genus *Nicotiana*.

9. The process of any of the Claims 6-8 wherein the species is *Nicotiana tabacum*.

10. A cytoplasmic male sterile plant produced by the process of any of the Claims 1-9.